# EUROPÄISCHE PATENTSCHRIFT

(11) **EP 0 667 134 B1**
(45) Veröffentlichungstag und Bekanntmachung des Hinweises auf die Patenterteilung: **19.05.1999**
(21) Anmeldenummer: 95100480.3
(22) Anmeldetag: 16.01.1995
(51) Int. Cl.: A61F 2/30, A61F 2/34, A61F 2/36, A61F 2/42

(54) **Orthopädisches Knochen-Implantat**
Orthopaedic bone implant
Implant osseux orthopédique

(30) Priorität: 15.02.1994 CH 439/94
(43) Veröffentlichungstag der Anmeldung: 16.08.1995
(73) Patentinhaber: Hermann, Werner, CH-6312 Steinhausen (CH)
(72) Erfinder: Knahr, Karl, Prof. Dr., A-1190 Wien (AT)
(74) Vertreter: OK pat AG

(56) Entgegenhaltungen:
- EP-A- 0 222 159
- EP-A- 0 282 789
- EP-A- 0 291 562
- EP-A- 0 318 679
- EP-A- 0 578 345
- FR-A- 2 622 791
- US-A- 5 147 386

## Beschreibung

Derartige Knochen-Implantate mit in die Knochensubstanz einschraubbaren Verankerungsteilen sind bereits in verschiedenartigen Ausführungen und Anwendungen bekannt, so z.B. bei Hüftgelenks-Prothesen. Dabei ist der genannte Rotationskörper beispielsweise eine Kugelkalotte oder ein Kegelstumpf, der mit der verjüngten Seite voran (dem "Polbereich" im Falle der Kalotte) in eine operativ vorbereitete Ausnehmung im Knochen eingesetzt wird. Ist nun das selbstschneidende Gewinde ein normales Spitzgewinde, so entstehen beim Eindrehen des Gewindes in die Knochensubstanz erhebliche Schwierigkeiten: Einerseits wird, wegen dem stetig zunehmenden Durchmesser der Gewindegänge, durch die Flanken des Gewindeprofils eine starke Keilwirkung auf den Knochen ausgeübt, was zu Knochenrissen führen kann. Zudem besteht zwischen den genannten Flanken und dem Knochen eine hohe, mit der im Eingriff befindlichen Gewindelänge zunehmende Reibung, was beim Eindrehen des Verankerungsteils bzw. des Implantats einen entsprechenden Kraftaufwand erfordert und den Knochen zusätzlich beansprucht. Hierbei ist zu bedenken, dass es praktisch unmöglich ist, beim selbstschneidenden Gewinde die Flanken der Schneidzähne im Gewindegang mit Freiwinkel herzustellen.

Bei einem bekannten Verankerungsteil eines Knochen-Implantats der oben genannten Gattung (Mantelhülse für die Gelenkspfanne einer Hüftgelenks-Prothese, EP-A-0 318 679) wurde versucht, gewisse beim Spitzgewinde auftretende Schwierigkeiten zu umgehen, indem die Mantelhülse mit einem Flachgewinde versehen wird. Mit dem dort dargestellten, relativ dünnen und hohen Flachgewindeprofil kann zwar die vorerwähnte Keilwirkung beim Eindrehen in das Hüftbein vermindert werden, jedoch bleibt die hohe Reibung an beiden Profil-Flachseiten bestehen. Ueberdies sind die Schneidzähne, wegen ihrer schmalen Basis zum Grundkörper, wenig widerstandsfähig gegen Seitenkräfte; es besteht deshalb die Gefahr, dass die Gewindestege vebogen werden, sei es bereits beim Einschrauben während der Operation oder später bei Belastung der Gelenkpfanne vom Femur-Gelenkkopf her. Bereits eine elastische Verbiegung der Gewindestege - und umso mehr natürlich bleibende Verbiegungen - können aber schwerwiegende medizinische Komplikationen verursachen.

Ferner ist aus der EP-A-0 291 562 eine Aussenschale für eine künstliche Hüftgelenkspfanne bekannt, welche eine Grundgestalt in Form eines Rotationskörpers und an dessen Aussenseite ein selbstschneidendes Gewinde besitzt, welches zum Einschrauben bzw. Verankern der Hüftgelenkspfanne in der Knochensubstanz bestimmt ist. Die das Gewinde tragende Rotationsfläche verjüngt sich in Achsrichtung, so dass sich der Gewindedurchmesser stetig verändert, wobei die Profile der Gewindegänge durch Teilhöhen eines spitzwinkligen Dreieckes gegeben sind. Die Abmessungen der Gewindegänge quer zur Achse verringern sich mit zunehmenden Abstand vom Polbereich bzw. proximalen Bereich zum Äquatorilalbereich bzw. distalen Bereich des Implantates. Dadurch entstehen im Polbereich tiefere Gewindegänge als im Äquatorialbereich, deren Querschnittsflächen, quer zu ihren Höhen betrachtet, aber etwa gleich sind. In einem die Achse enthaltenden Schnitt der Aussenschale ergibt also das über die Grundgestalt hinausragende Material Vorsprünge, die im proximalen Bereich lang und im distalen Bereich kurz sind, deren Stärke aber in allen Bereichen etwa gleich ist, derart, dass das Verhältnis von Länge zu Stärke im proximalen Bereich bedeutend grösser ist als im distalen Bereich.

Mit der vorliegenden Erfindung wird angestrebt, durch geeignete Gestaltung des selbstschneidenden Gewindes die erwähnten Nachteile bekannter Knochen-Implantate der eingangs genannten Art zu überwinden. Vor allem soll bei hoher Stabilität des Gewindes die Reibung beim Einschrauben wie auch die Keilwirkung auf die umgebende Knochensubstanz entscheidend herabgesetzt werden.

Diese Aufgabe wird erfindungsgemäss durch die Merkmale des kennzeichneden Teils des Patentanspruchs 1 gelöst.

Wie weiter unten näher ausgeführt, lässt sich die Aussenkontur des Verankerungsteils mit solchem Gewinde insbesondere auf numerisch gesteuerten Dreh- und/oder Fräsmaschinen durchaus herstellen. Mit dem erfindungsgemässen Gewindeprofil wird auch der besondere Vorteil erzielt, dass die Bereiche der Gewindegänge mit grösserem Profilquerschnitt mehr ins Innere des Knochens zu liegen kommen, dagegen die "schmaleren" Bereiche an den Rand der Ausnehmung im Knochen.

Besondere und vorteilhafte Ausgestaltungen und Anwendungen des erfindungsgemässen Knochen-Implantats sind in den abhängigen Ansprüchen definiert. Verschiedene Ausführungsbeispiele des Erfindungsgegenstandes werden nachstehend in Verbindung mit der Zeichnung näher beschrieben.
- Fig. 1: zeigt als erstes Ausführungsbeispiel die Stirnansicht einer Mantelhülse zur Aufnahme der Gelenkspfanne einer Hüftgelenks-Prothese,
- Fig. 2: zeigt die Mantelhülse nach Fig. 1 halb in Seitenansicht und halb geschnitten, bei Beginn des Einschraubens in den Beckenknochen,
- Fig. 3: zeigt schematisch verschiedene, von einem spitzwinkligen Dreieck abgeleitete Querschnitte entlang eines Gewindeganges in Abwicklung,
- Fig. 4: veranschaulicht ebenfalls schematisch (nicht massstäblich) den fortschreitenden Schneidvorgang durch aufeinanderfolgende Zähne des selbstschneidenden Gewindes,
- Fig. 5: zeigt als zweites Ausführungsbeispiel einen Femurschaft einer Hüftgelenks-Prothese in Ansicht, und
- Fig. 6: veranschaulicht, ebenfalls in Seitenansicht, als drittes Ausführungsbeispiel eine Fingergelenk-Prothese.

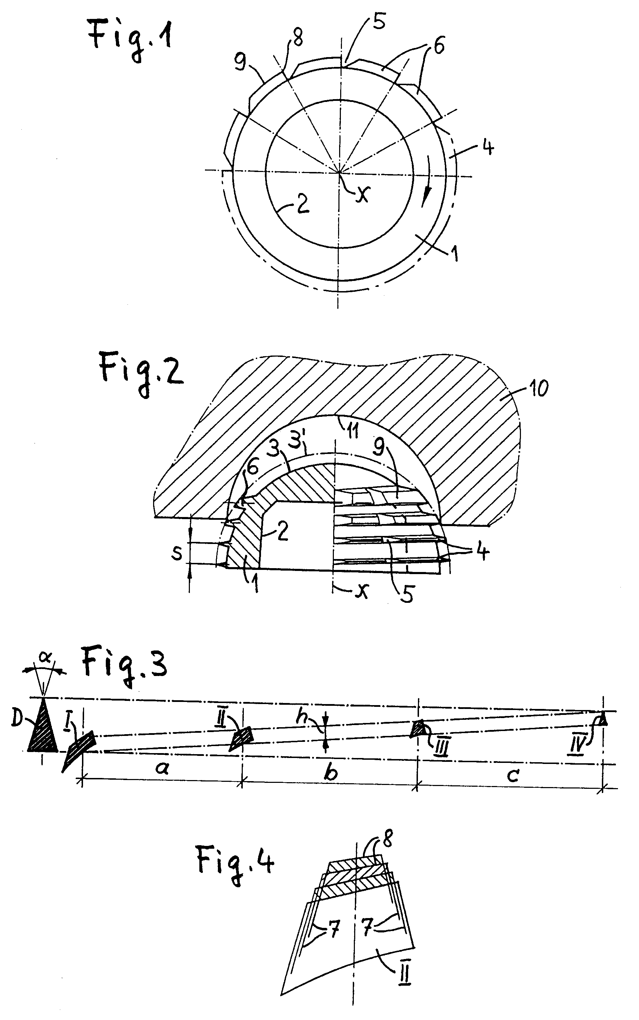

Die zur Aufnahme der Gelenkspfanne einer Hüftgelenksprothese bestimmte Mantelhülse 1 nach Fig. 1 und 2 ist im wesentlichen als Rotationskörper mit der Achse x gestaltet. Die nicht dargestellte Gelenkspfanne selbst wird in eine Ausnehmung 2 der Mantelhülse 1 eingesetzt. Die Rotationsfläche (Aussenfläche) der Hülse 1 ist in Richtung der Achse x verjüngt; es kann sich dabei, wie dargestellt, um eine Kugelfläche 3 oder, wie an sich ebenfalls bekannt, um eine abgestumpfte Kegelfläche handeln.

Im Beckenknochen 10 (Hüftbein), in den die Mantelhülse 1 eingesetzt werden soll, wird bei der Hüftgelenksoperation eine Ausnehmung 11 vorbereitet, die möglichst der Rotationsfläche 3 entspricht. Um die Mantelhülse einschrauben und im Knochen verankern zu können, weist sie an ihrer Aussenseite ein selbstschneidendes Gewinde 4 auf. Der Gewindedurchmesser ändert sich dabei stetig entsprechend der Verjüngung der das Gewinde tragenden Rotationsfläche 3. Das Gewinde 4 lässt jedoch den der Ausnehmung 11 zugekehrten Polbereich der Kugelkalotte frei. Anstelle des dargestellten eingängigen Gewindes sind auch mehrgängige Gewinde denkbar.

Um die Schneidzähne 6 des selbstschneidenden Gewindes 4 zu bilden, sind die Gewindegänge durch Einfräsungen 5 unterbrochen, die vorzugsweise entlang von Mantellinien (Meridianen) des Rotationskörpers geführt sind. Die Stirnflächen der Schneidzähne 6 können selbstverständlich (entgegen der Darstellung in Fig. 1) einen Spanwinkel aufweisen. Wie aus Fig. 2 hervorgeht, liegen die äusseren Schneidkanten 8 der Zähne 6 zweckmässigerweise auf einer Rotationsfläche 3', die zur Rotationsfläche 3 konzentrisch liegt, jedoch ist dies nicht unbedingt erforderlich. Auch können die anschliessenden Aussenflächen 9 der Zähne 6 zur Bildung eines Freiwinkels bearbeitet werden.

Wesentlich am vorliegenden selbstschneidenden Gewinde 4 ist das Profil der Gewindegänge derart, dass mit zunehmendem Gewindedurchmesser (bzw. -Radius) der Profilquerschnitt stetig abnimmt. Dieser Sachverhalt - und die geometrische Bestimmung aufeinanderfolgender Querschnitte - wird nachstehend anhand der Fig. 3 erläutert.

Es sind vier Querschnitte bzw. Gewindegang-Profile I - IV entlang der gedachten Abwicklung eines Gewindeganges dargestellt, die auf einem bestimmten Meridian der Rotationsfläche 3 und auf dem entsprechenden, zunehmenden Radius (Abstand zur Achse x) liegen; angenommen sind durch Ausfräsungen 5 gebildete Schneidflächen (Stirnflächen) von Zähnen 6. Die Mittenabstände a, b, c der Querschnitte entsprechen demnach dem jeweiligen (zunehmenden) Umfang eines Gewinde-Umganges. Die Basislinie der Querschnitte I - IV ist jeweils durch die Rotationsfläche 3 gegeben und die Deckenlinie (gegebenenfalls) durch die äussere Rotationsfläche 3'. Dabei ist die Höhe h zweckmässigerweise praktisch konstant gehalten. Wie aus Fig. 3 weiter ersichtlich ist, handelt es sich bei den Profilen I - IV (und allen zwischenliegenden Profilen) um Teilhöhen bzw. Teilausschnitte eines spitzwinkligen Dreiecks D, wobei diese Teilhöhen sich bei zunehmendem Durchmesser des Gewindeganges von der Basis des Dreiecks gegen dessen Spitze hin verschieben. Somit sind bei gegebener "Höhenlage" (Radius) die Seitenflanken der Querschnitte und damit die Querschnitte überhaupt - durch das Dreieck D gegeben.

In der Fig. 3 ist der besseren Anschaulichkeit wegen ein Spitzenwinkel α des Dreiecks D von etwa 30°gewählt worden. Der Spitzenwinkel sollte in der praktischen Ausführung in einem Bereich von etwa 5° bis 35° liegen (wobei eine starke Durchmesser-Aenderung der Rotationsfläche spitzere Winkel erfordert, "schlanke" Rotationskörper dagegen stumpfere Winkel zulassen). Das beschriebene Gewinde lässt sich z.B. auf einer numerisch gesteuerten Drehmaschine dadurch herstellen, dass die Steigung der einen Seitenflanke des Gewindeganges etwas geringer und die Steigung der anderen Seitenflanke etwas grösser eingestellt (programmiert) wird als die "nominelle" Gewindesteigung (selbstverständlich muss dabei auch der variable Durchmesser programmiert werden). Der Abstand s zwischen aufeinanderfolgenden Gewinde-Umgängen wird zweckmässigerweise so gross bemessen, dass dazwischen sich die Rotationsfläche 3 fortsetzt. Es ergibt sich dadurch - bei im Beckenknochen 10 festgezogener Mantelhülse 1 - eine vorteilhafte Vergrösserung der Auflagefläche und entsprechend verbesserte Last-Einleitung in den Knochen.

Das Dreieck D, das wie vorstehend beschrieben das Gewindegang-Profil bestimmt, muss nicht unbedingt (wie in Fig. 3 dargestellt) gleichschenklig bzw. "symmetrisch" sein, sondern es kann sich auch um ein ungleichschenkliges, d.h. schief auf der Basis stehendes Dreieck handeln. Dabei ergibt sich dann ein Profil, dessen Seitenflanken zu einer Normalebene zur Achse x ungleiche Winkel einnehmen; beispielsweise kann man auf diese Weise eine Art Sägezahnprofil erreichen. Ebenfalls ist es nicht Bedingung, dass die Profil-Teilhöhe h sich bis zur Spitze des Dreiecks D verschiebt, dass also der Gewindegang (wie in Fig. 2 und 3 gezeigt) am Ende spitz ausläuft.

Das durch Teilhöhen eines spitzwinkligen Dreiecks D definierte Gewinde 4 kann anschaulich und näherungsweise als stetiger Uebergang von einem (breiten) Trapezgewinde in ein (schmales) Spitzgewinde aufgefasst werden (wobei, wie erwähnt, nicht notwendigerweise am Ende des Gewindeganges die Spitze erreicht werden muss). Die sich daraus ergebenden besonderen Vorteile lassen sich anhand der schematischen, nicht massstäblichen Darstellung nach Fig. 4 erkennen. Ausgehend etwa vom Querschnitt II (Fig. 3) sind schematisch weitere Querschnitte bzw. Schneidzähne 6 dargestellt, die beim Schneiden des Gewindes aufeinanderfolgen. Von solchen Zähnen werden im bereits geschnittenen "Kanal" jeweils die schraffierten Teilbereiche ausgeschnitten, wobei der Schnitt entsprechend dem zunehmenden Radius praktisch nur an den radial aussenliegenden Schneidkanten 8 (Fig. 1) erfolgt. Dagegen bleiben die Seitenflanken 7 der (immer schmaler werdenden) Schneidzähne frei, d.h. sie haben praktisch keine Reibung an der umgebenden Knochensubstanz; ebensowenig üben sie eine Keilwirkung auf den Knochen aus. Jedoch ist eine ausgezeichnete Führung des Gewindesteges im "Kanal" während des Schneidens und bis zum Festziehen (Auflage zwischen den Flächen 3 und 11, Fig. 2) dennoch gegeben, weil der Steg, zwar zunehmend schmaler werdend, sich stetig radial ausdehnt und sich gewissermassen in den Kanal hineinschiebt (Fig. 4). Damit ist von Anfang an ein satter Sitz der Mantelhülse und eine relativ tragfähige Verbindung zum Knochen gewährleistet.

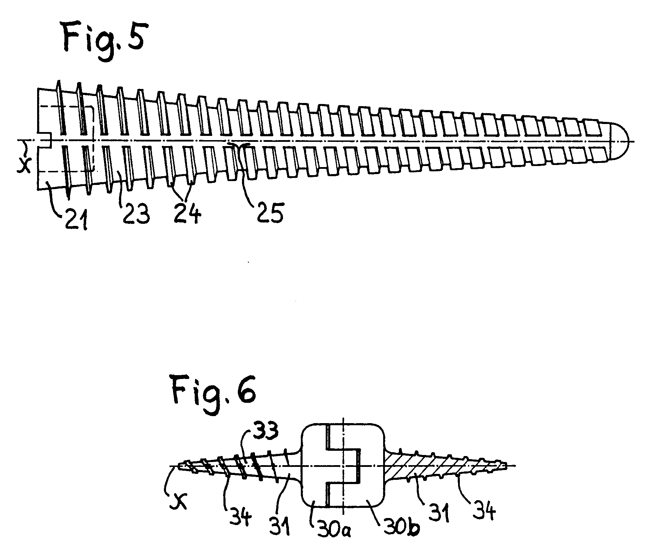

Als weiteres Ausführungsbeispiel eines Knochen-Implantats nach der Erfindung ist in Fig. 5 ein Femurschaft 21 einer Hüftgelenks-Prothese dargestellt. Ein solcher Femurschaft wird axial in den Oberschenkelknochen (Femur) eingeschraubt und wird (über die links in Fig. 5 gestrichelt angedeutete stirnseitige Ausnehmung) mit der Gelenkkugel der Prothese verbunden. Der schlanke, verjüngte Schaft 21 ist als Verankerungsteil mit einem selbstschneidenden Gewinde 24 versehen, das in analoger Weise wie oben beschrieben gestaltet ist. Mit 25 sind die Einfräsungen in Schaft-Längsrichtung zur Bildung der Schneidzähne bezeichnet, und mit 23 die das Gewinde 24 tragende Rotationsfläche. Im vorliegenden Fall, wo es sich um einen langestreckten Verankerungsteil mit zahlreichen Gewindeumgängen handelt, ist es zweckmässig, nicht gleichbleibende Teilhöhen h (Fig. 3) vorzusehen, sondern die Teilhöhen am Anfang (dünneres Schaftende, rechts in Fig. 5) kleiner zu bemessen und mit zunehmendem Schaftdurchmesser grösser werden zu lassen (wie in Fig. 5 deutlich sichtbar).

Die Fig. 6 zeigt als weiteres Ausführungs- und Anwendungsbeispiel eine Fingergelenk-Prothese als Knochen-Implantat. Die beiden Gelenkhälften 30a und 30b weisen als Verankerungsteil je einen konischen Schaft 31 mit der Konusfläche 33 und darauf angebrachtem, selbstschneidendem Gewinde 34 auf. Ueber die Gestaltung dieses Gewindes und die damit erzielten Eigenschaften und Vorteile gilt das weiter oben Gesagte.

## Patentansprüche

1. Orthopädisches Knochen-Implantat mit einem Verankerungsteil (1, 21, 31) in der Grundgestalt eines Rotationskörpers, der an seiner Aussenseite ein selbstschneidendes, zum Einschrauben und Verankern in der Knochensubstanz (10) bestimmtes Gewinde (4, 24, 34) trägt, wobei die das Gewinde tragende Rotationsfläche (3, 23, 33) in Achsrichtung verjüngt ist und der Gewindedurchmesser sich entsprechend stetig ändert, wobei das Gewindegang-Profil (I-IV) durch Teilhöhen (h) eines spitzwinkligen Dreiecks (D) gegeben ist, dadurch **gekennzeichnet**, dass die Teilhöhen (h) sich, in Richtung zunehmenden Durchmessers gesehen, von der Basis zur Spitze des Dreiecks (D) hin verschieben, wobei der Profilquerschnitt entsprechend stetig abnimmt.

2. Knochen-Implantat nach Anspruch 1, dadurch gekennzeichnet, dass der Spitzenwinkel (α) des Dreiecks (D) etwa 5° bis 35° beträgt.

3. Knochen-Implantat nach Anspruch 1, dadurch gekennzeichnet, dass die Teilhöhen (h) entlang dem Gewindegang mindestens angenähert gleich bleiben.

4. Knochen-Implantat nach Anspruch 1, dadurch gekennzeichnet, dass der Abstand (s) zwischen aufeinanderfolgenden Umgängen des Gewindes (4, 24, 34) so gross bemessen ist, dass die Rotationsfläche (3, 23, 33) sich zwischen den Gewindegängen fortsetzt.

5. Knochen-Implantat nach einem der vorangehenden Ansprüche, dadurch gekennzeichnet, dass der Verankerungsteil (1) eine Mantelhülse zur Aufnahme der Gelenkspfanne einer Hüftgelenks-Prothese ist.

6. Knochen-Implantat nach einem der Ansprüche 1 bis 4, dadurch gekennzeichnet, dass der Verankerungsteil (21) ein Femurschaft zur Aufnahme der Gelenkskugel einer Hüftgelenks-Prothese ist.

7. Knochen-Implantat nach einem der Ansprüche 1 bis 4, dadurch gekennzeichnet, dass der Verankerungsteil (31) ein Gewindeschaft einer Fingergelenk-Prothese ist.

## Claims

1. An orthopaedic bone implant having an anchoring part (1, 21, 31) in the fundamental form of a body of revolution which bears on its outside a self-cutting screw thread (4, 24, 34) which is intended for screwing into and anchoring in the bone substance (10), wherein the surface of revolution (3, 23, 33) which bears the screw thread is tapered axially and the screw thread diameter correspondingly changes continuously, wherein the thread turn profile (I-IV) is determined by sectional heights (h) of an acute-angled triangle (D), characterised in that as seen in the direction of increasing diameter the sectional heights (h) are displaced from the base to the apex of the triangle (D), wherein the profile cross-section correspondingly decreases continuously.

2. A bone implant according to claim 1, characterised in that the apex angle (α) of the triangle (D) is about 5° to 35°.

3. A bone implant according to claim 1, characterised in that the sectional heights (h) remain at least approximately equal along the screw thread.

4. A bone implant according to claim 1, characterised in that the pitch (s) between successive turns of the screw thread (4, 24, 34) is designed with a magnitude such that the surface of revolution (3, 23, 33) continues between the screw thread turns.

5. A bone implant according to any one of the preceding claims, characterised in that the anchoring part (1) is a casing sleeve for receiving the joint socket of a hip joint prosthesis.

6. A bone implant according to any one of claims 1 to 4, characterised in that the anchoring part (21) is a femur shank for receiving the joint ball of a hip joint prosthesis.

7. A bone implant according to any one of claims 1 to 4, characterised in that the anchoring part (31) is a threaded shank of a finger joint prosthesis.

## Revendications

1. Implant osseux orthopédique comportant une partie d'ancrage (1,21,31) possédant la configuration de base d'un corps de révolution, qui porte, sur sa face extérieure, un filetage autotaraudeur, destiné à réaliser le vissage et l'ancrage dans la substance osseuse (10), et dans lequel la surface de révolution (3,23,33), qui porte le filetage, se rétrécit dans la direction axiale et le diamètre du filetage varie continûment de façon correspondante, et dans lequel le profil (I-IV) de la spire de filetage est défini par des hauteurs partielles (h) d'un triangle acutangle (D), caractérisé en ce que lorsqu'on regarde dans la direction de diamètres croissants, les hauteurs partielles (h) se décalent de la base vers le sommet du triangle (D), la section transversale du profil diminuant continûment de façon correspondante.

2. Implant osseux selon la revendication 1, caractérisé en ce que l'angle au sommet (α) du triangle (D) est compris entre environ 5° et 35°.

3. Implant osseux selon la revendication 1, caractérisé en ce que les hauteurs partielles (h) restent au moins approximativement constantes le long de la spire de filetage.

4. Implant osseux selon la revendication 1, caractérisé en ce que la distance (s) entre des circonvolutions successives du filetage (4,24,34) est dimensionnée de telle sorte que la surface de révolution (3,23,33) se prolonge entre les spires du filetage.

5. Implant osseux selon l'une des revendications précédentes, caractérisé en ce que la partie d'ancrage (1) est une douille-enveloppe servant à loger la cavité articulaire d'une prothèse d'articulation de la hanche.

6. Implant osseux selon l'une des revendications 1 à 4, caractérisée en ce que la partie d'ancrage (21) est une broche de fémur destinée à recevoir l'articulation sphérique d'une prothèse de la hanche.

7. Implant osseux selon l'une des revendications 1 à 4, caractérisé en ce que la partie d'ancrage (31) est une tige filetée d'une prothèse de l'articulation d'un doigt.
